# EUROPÄISCHE PATENTSCHRIFT

(11) **EP 0 530 343 B1**
(45) Veröffentlichungstag und Bekanntmachung des Hinweises auf die Patenterteilung: **20.09.1995**
(21) Anmeldenummer: 92907030.8
(22) Anmeldetag: 19.03.1992
(51) Int. Cl.: C07C 237/46, C07D 317/24, A61K 49/04

(54) **WASSERLÖSLICHE NICHT IONISCHE RÖNTGENKONTRASTMITTEL UND VERFAHREN ZU IHRER HERSTELLUNG**
WATER SOLUBLE, NON-IONIC X-RAY CONTRASTING AGENTS AND PROCESS FOR PRODUCING THE SAME
AGENTS CONTRASTANTS RADIOGRAPHIQUES NON IONIQUES SOLUBLES DANS L'EAU ET LEUR PROCEDE DE PREPARATION

(30) Priorität: 20.03.1991 DE 4109169
(43) Veröffentlichungstag der Anmeldung: 10.03.1993
(73) Patentinhaber: Dr. Franz Köhler Chemie GmbH, D-64665 Alsbach-Hähnlein (DE)
(72) Erfinder: BRUNS, Wilfried, D-6144 Zwingenberg (DE); KÖHLER, Gernot, D-6146 Alsbach (DE)
(74) Vertreter: Zinngrebe, Horst, Dr.rer.nat.
(86) Internationale Anmeldenummer: DE9200237
(87) Internationale Veröffentlichungsnummer: WO9216498

(56) Entgegenhaltungen:
- EP-A- 0 173 624
- WO-A-87/00757
- DE-A- 2 720 832
- DE-A- 3 138 397
- FR-A- 2 385 698
- US-A- 3 946 045
- US-A- 4 288 629
- US-A- 4 310 700
- CHEMICAL ABSTRACTS, Band 81, Nr. 14, 3. April 1978, Columbus, Ohio, US; Zusammenfassung Nr. 90971s, V.V. BEZUGLOV: "Sythesis of two analogs of phosphatidylchloline", Seite 417
- JOURNAL OF ORGANIC CHEMISTRY, Bd. 52, Nr. 5, 6. März 1987, Washington, US; Seiten 819 - 827; S.P.TANIS et al: "Pyrroles as terminators in cationic cyclisations. The preparation of 5,6,7,8-tetrahydroindolizidines and 6,7,8,9-tetrahydro-5H-pyrrolo[1,2-A]azepines"
- JOURNAL OF ORGANIC CHEMISTRY, Bd. 37, Nr. 13, 30. Juni 1972, Washington, US; Seiten 2197 - 2201; V.W. GASH: "Dineoalkyl ethers. A general synthesis of the symmetrical ethers"
- JOURNAL OF HETEROCYCLIC CHEMISTRY, Bd. 26, Nr. 5, September 1989, Provo, US; Seiten 1261 - 1271; J. HANNA et al: "Carba-acylnucleoside antiherpetic agents"
- HELVETICA CHIMICA ACTA, Bd. 31, Nr. 2, 8. März 1978, Basel, CH; Seiten 885 - 898; P.C. WÄLCHLI et al: "Synthese der vier stereoisomeren Dihydropalustraminsäuren"

## Beschreibung

Die Erfindung umfaßt neue, sehr gut wasserlösliche, nicht ionische Röntgenkontrastmittel mit niedrigem osmotischem Druck. Diese Verbindungen werden durch die allgemeinen Formeln I, II, III oder IV dargestellt: 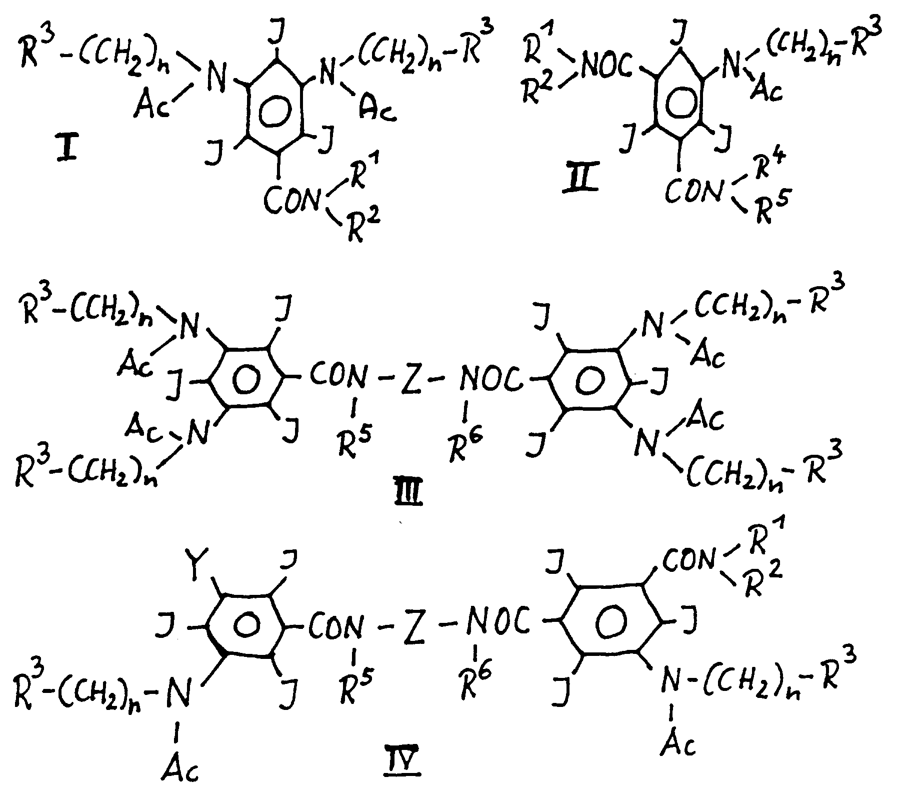
worin Ac einen Acyl-, Hydroxyacyl, Polyhydroxyacyl- oder Alkoxyacylrest mit 1 bis 4 Kohlenstoffatomen bedeutet; z. B. Acetyl, Propionyl, 2-Hydroxyacetyl, 2-Methoxyacetyl oder 2-Hydroxypropionyl. R1, R2, R4, R5 und R6 symbolisieren Wasserstoff einen Alkyl, Hydroxyalkyl, Polyhydroxyalkylrest mit 1 bis 4 Kohlenstoffatomen wie z. B. Methyl, Ethyl, Propyl, Butyl, 2-Hydroxyethyl, 2,3-Dihydroxypropyl, 1,3-Dihydroxyisopropyl.

R3 stellt entweder einen linearen Hydroxyalkyl-, Polyhydroxyalkyl-, Hydroxyalkoxy-, oder Polyhydroxyalkoxyrest mit 1 bis 4 Kohlenstoffatomen wie z. B. Hydroxymethyl, 1,2-Dihydroxyethyl, 1,2,3-Trihydroxypropyl, 2-Hydroxyethoxy, 2,3-Dihydroxypropoxy dar; dann liegt n im Bereich 2 bis 4. R3 kann auch einen verzweigten Polyhydroxyalkylrest mit 3 bis 6 Kohlenstoffatomen darstellen wie 1,3-Dihydroxyisopropyl, 2-Hydroxymethyl-1-3-dihydroxyisopropyl oder 1-Hydroxymethyl-2,3-dihydroxypropyl, dann nimmt n die Werte 1 bis 3 an; wobei zwischen dem acylierten Stickstoffatom und dem ersten Kohlenstoffatom im Rest R3, das eine Hydroxylgruppe trägt, sich mindestens zwei Kohlenstoffatome befinden, die keine Hydroxylgruppe tragen.

Z bedeutet eine direkte Bindung der Stickstoffatome (Hydrazinderivat) oder eine Alkylenkette mit 2 bis 6 Kohlenstoffatomen, die gegebenenfalls durch Sauerstoff unterbrochen sind.

Y symbolisiert eine der beiden folgenden Gruppen 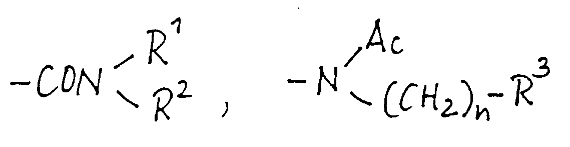
worin R1, R2, R3, n und Ac die oben beschriebene Bedeutung haben.

Die hier beschriebenen Verbindungen unterscheiden sich von bekannten, vergleichbaren Verbindungen dadurch, daß zwischen dem acylierten Stickstoffatom und dem ersten Kohlenstoffatom im Rest R3, das eine Hydroxylgruppe trägt, sich mindestens zwei Kohlenstoffatome befinden, die keine Hydroxylgruppe tragen.

Dieses führt zu einer unerwarteten Absenkung der Osmolalität und zu einer unerwarteten Steigerung der Löslichkeit bei gleicher Zahl der Hydroxylgruppen.

Einige zentrale Forderungen an universell verwendbare Röntgenkontrastmittel sind hohe Wasserlöslichkeit, geringer osmotischer Druck und nicht zu hohe Viskosität. Diese Forderungen werden von vielen der erfindungsgemäßen Verbindungen in hervoragender Weise erfüllt. Bei allen in den Ansprüchen namentlich benannten Verbindungen liegt die Löslichkeit über 90% (w/v). Die Lösungen der erfindungsgemäßen Verbindungen weisen einen ungewöhnlich niedrigen osmotischen Druck auf. Alle derzeit im Handel befindlichen monomeren, nicht ionischen Röntgenkontrastmittel besitzen bei 300 mg I/ml Osmolalitäten zwischen 600 und 700 mOsmol/kg. Eine Ausnahme ist hier lediglich das Metrizamid mit 485 mOsmol/kg, es weist jedoch schwerwiegende andere Nachteile auf. Die erfindugnsgemäßen Verbindungen entsprechend den allgemeinen Formeln I bis IV zeigen bei 300 mg I/ml Osmolalitäten zwischen 215 und 545 mOsmol/kg. Die Viskositäten entsprechen mit wenigen Ausnahmen denen der zur Zeit im Handel befindlichen nichtionischen Röntgengenkontrastmittel.

Die erfindungsgemäßen Verbindungen entsprechend den allgemeinen Formeln I bis IV sind in Lösung stabil, so daß sie durch erhitzen auf 120°C bei physiologischem pH-Wert sterilisiert werden können.

Aus EP-A-406992 sind zwar nichtionische Carboxamid-Kontrastmittel bekannt, die bei niedriger Osmolalität eine brauchbare Viscosität zeigen. Jedoch sind die Methoden zur Darstellung dieser Verbindungen aufwendig und teuer. Demgegenüber bietet die Erfindung einfachere und preiswertere Alternativen und zeigt darüber hinaus die die Osmolalität senkende Wirkung der Gruppen (CH₂)ₙ in den verlängerten Seitenketten sowie gegebenenfalls weiterer Substituenten, wie beispielsweise eine zunehmende Alkylierung des Carboxamidstickstoffs. In der nachfolgenden Tabelle beziehen sich die römischen Nummern der aufgeführten Verbindungen auf diejenigen gleicher Nummer, die in den Ansprüchen angegeben sind.

| Verbindg | Molmasse | Iodgehalt %I | Löslichkeit %(w/v) | Osmolalität mOsmol/kg | Viskosität cps |
|---|---|---|---|---|---|
| VI | 803,16 | 47,4 | >100 | 545 | 5,3 |
| VII | 817,19 | 46,6 | >100 | 535 | 4,5 |
| VIII | 831,21 | 45,8 | >100 | 330 | 7,5 |
| IX | 845,24 | 45,0 | >100 | 500 | 6,3 |
| XII | 861,24 | 44,2 | >100 | 495 | 8,2 |
| XIX | 789,13 | 48,2 | >100 | 405 | 5,8 |
| XXX | 845,24 | 45,0 | >100 | 285 | 8,7 |
| XXXI | 859,27 | 44,3 | >100 | 215 | 17,0 |
| XXXIII | 817,19 | 46,6 | >100 | 365 | 6,7 |
| XXXIV | 831,21 | 45,8 | >100 | 255 | 8,4 |
| XXXVI | 859,27 | 44,3 | >100 | 285 | 17,0 |
| XXXVII | 873,29 | 43,6 | >100 | 510 | 12,5 |
| XIV | 1688,46 | 45,1 | >100 | 300 | ca. 19 |
| XV | 1632,35 | 46,6 | >100 | 255 | 16,5 |
| XVI | 1648,35 | 46,2 | >100 | 525 | 11,0 |

Die Osmolalität und Viskosität beziehen sich auf Lösungen in Wasser mit 300 mg I/ml bei 37°C.

Die Erfindung beinhaltet folgendes Verfahren zur Herstellung von Verbindungen der allgemeinen Formeln I bis IV.

Ein reaktives Säurederivat, bevorzugt das Säurechlorid, einer 3,5-Diacylamido-2,4,6-triiodbenzoesäure oder eines 5-Acylamido-2,4,6-triiodisophthalsäurederivats wird in einem geeignetem Lösungsmittel wie z. B. Dimethylformamid, Dimethylacetamid, Tetrahydrofuran, Dioxan, Acetonitril mit einem primären oder sekundären Amin umgesetzt. Zur Herstellung dimerer Kontrastmittel entsprechend den Formeln III und IV werden Diamine, bevorzugt Derivate des Ethylendiamins, eingesetzt.

Das erhaltene Amid wird in Alkalilauge gelöst, mit einem als Lösungsvermittler geeignetem Lösungsmittel wie z. B. niedere Alkohole, Dimethylformamid, Dimethylacetamid, Tetrahydrofuran, Dioxan, Acetonitril, Glycolether, Diglycolether versetzt und mit einem Alkylierungsmittel der allgemeinen Formel V bei Temperaturen zwischen 10° und 90°C umgesetzt:

X-(CH₂)ₙ-R³ V

worin X ein Halogen, einen Sulfonsäureester oder eine andere gebräuchliche Austrittsgruppe Nukleofug bedeutet.

R3 stellt entweder einen linearen Hydroxyalkyl, Polyhydroxyalkyl, Hydroxyalkoxy, oder Polyhydroxyalkoxyrest mit 1 bis 4 Kohlenstoffatomen wie z. B. Hydroxymethyl, 1,2-Dihydroxyethyl, 1,2,3-Trihydroxypropyl, 2-Hydroxyethoxy, 2,3-Dihydroxypropoxy dar; dann liegt n im Bereich 2 bis 4. R3 kann auch einen verzweigten Polyhydroxyalkylrest mit 3 bis 6 Kohlenstoffatomen darstellen wie 1,3-Dihydroxyisopropyl, 2-Hydroxymethyl-1,3-dihydroxyisopropyl oder 1-Hydroxymethyl-2,3-dihydroxypropyl, dann nimmt n die Werte 1 bis 3 an. Entscheidend ist, daß zwischen der Austrittsgruppe X und dem ersten Kohlenstoffatom im Rest R3, das eine Hydroxylgruppe trägt, sich mindestens zwei Kohlenstoffatome befinden, die keine Hydroxylgruppe tragen.

Die Hydroxylgruppen des Restes R3 sind in der Regel durch geeignete Schutzgruppen, bevorzugt als Acetale oder Ketale blockiert.

Die Synthese wird meist durch eine Deblockierung geschützter Hydroxylgruppen abgeschlossen.

Alternativ kann bei der Herstellung von Verbindungen entsprechend der allgemeinenen Formeln I bis IV auch die Reaktionsfolge Amidierung Alkylierung vertauscht sein; d. h. zunächst wird 3,5-Diacylamido-2,4,6-triiodbenzoesäure, 5-Acylamido-2,4,6-triiodisophthalsäure oder ein 5-Acylamidotriiodisophthalsäuremonoamid mit einem Alkylierungsmittel entsprechend Formel V in Gegenwart einer geeigneten Base umgesetzt. Erst jetzt erfolgt die Amidierung noch freier Carboxylgruppen durch Überführung ins Säurechlorid und anschließender Umsetzung mit einem Amin der folgenden Formel 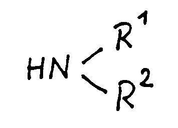
wobei R1, R2 die oben beschriebene Bedeutung besitzen. Diese Vorgehensweise kann es erforderlich machen, daß nach der Alkylierung säurelabile Hydoxylschutzgruppen (Acetale,Ketale) durch hinreichend säurestabile Schutzgruppen (Ester) ersetzt werden.

Der Abschluß der Synthese erfolgt auch hier durch eine Abspaltung aller eventuell vorhandenen Schutzgruppen.

### Beispiele

### Beispiel 1

### 1,2-O-Isopropyliden-5-O-p-toluolsulfonyl-1,2,5-pentantriol

### A. 1,2-O-Isopropyliden-1,2,5-pentantriol

Ein Gemisch aus 1,2,5-Pentantriol (50g) und Aceton (500ml) wird nach Zugabe von p-Toluolsulfonsäure (0,1g) 20 Stunden kräftig bei Raumtemperatur gerührt. Nun gibt man konzentrierter Amoniak (5ml) zu und engt ein. Der Rückstand wird in Ether aufgenommen und mit Kochsalzlösung gewaschen. Die Etherphase wird über Natriumsulfat getrocknet und eingeengt. Farbloses Öl (52g = 78% d. Th.)

### B. 1,2-O-Isopropyliden-5-O-p-toluolsulfonyl-1,2,5-pen tantriol

1,2-O-Isopropyliden-1,2,5-pentantriol (50g) werden in Acetonitril gelöst, man gibt Triethylamin (140ml) zu und kühlt auf 0°C. Nun wird p-Toluolsulfonyl-chlorid (65g) zugegeben. Das Reaktionsgemisch wird 20 Stunden gerührt wobei es sich langsam auf Raumtemperatur erwärmt. Es wird eingeengt, mit Wasser (400ml) versetzt und mehrmals mit t-Butylmethylether extrahiert. Nach dem Trocknen und Einengen der Etherphasen wird ein farbloses Öl erhalten. (84g = 86% d. Th.)

Auf analoge Weise wird aus 1,2,4-Butantriol das 1,2-O-Isopropyliden-4-O-p-toluolsulfonyl-1,2,4-butantriol erhalten.

### Beispiel 2

### 3,5-Bis(N-(3,4-dihydroxybutyl)-acetamido)-2,4,6-triiodbenzoesäure-N-methylamid VI

### A. 3,5-Diacetamido-2,4,6-triiodbenzoesäure-N-methylamid

Zu einer Lösung von Diatrizoylchlorid (130g) in Dimethylformamid (1l) wird Methylammoniumchlorid (20g) und Triethylamin (83ml) gegeben. Dieses Gemisch wird 5 Stunden bei 50°C gerührt. Nun wird eingeengt, der Rückstand in Wasser suspendiert und abfiltriert.

Der Rückstand wird mit 1N Salzsäure, mit Wasser, mit Natriumhydrogencarbonatlösung und wieder mit Wasser gewaschen. Nach dem Trocknen wird ein weißer Festkörper erhalten. (110g = 87% d. Th.)

### B. 3,5-Bis-(N-(3,4-O-isopropyliden-3,4-dihydroxybutyl)-acet-amido)-2,4,6-triiodbenzoesäure-N-methylamid

3,5-Diacetamido-2,4,6-triiodbenzoesäure-N-methylamid (100g) werden in 2N Natronlauge (400ml) gelöst. Man gibt Tetrahydrofuran (500ml) und 1,2-O-Isopropyliden-4-O-p-toluolsulfonyl-1,2,4-butan-triol (125g) zu. Das Reaktionsgemisch wird 60 Stunden bei 60° bis 70°C gerührt. Nach dem Abkühlen wird eingeengt, mit Wasser versetzt und mehrmals mit Ethylacetat extrahiert. Die organischen Phasen werden getrocknet, eingengt und in Ethylacetat über Kieselgel chromatographiert. (104g = 73% d.Th.)

### C. 3,5-Bis-(N-(3,4-dihydroxybutyl)-acetamido)-2,4,6-triiodbenzoesäure-N-methylamid VI

Man löst 3,5-Bis-(N-(3,4-O-isopropyliden-3,4-dihydroxybutyl)acetamido)-2,4,6-triiodbenzoesäure-N-methylamid (50g) in Methanol (200ml) gibt 1N Salzsäure (20ml) und Wasser (50ml) zu und läßt 20 Stunden bei Raumtemperatur stehen. Nun wird eingeengt und in Wasser aufgenommen. Die erhaltene Lösung wird über Kohle filtriert und anschließend über eine Anionentauschersäule (200ml Dowex 1 X 2 OH-Form) entsäuert. Nach dem Einengen wird ein weißer Feststoff erhalten. (43g = 95% d. Th.)

### Beispiel 3

### 3,5-Bis(N-(3,4-dihydroxybutyl)-acetamido)-2,4,6-triiodbenzoesäure-N,N-dimethylamid VII

### A. 3,5-Diacetamido-2,4,6-triiodbenzoesäure-N,N-dimethylamid

Zu einer Lösung von Diatrizoylchlorid (130g) in Dimethylformamid (1l) wird Dimethylammoniumchlorid (25g) und Triethylamin (83ml) gegeben. Dieses Gemisch wird 5 Stunden bei 50°C gerührt. Nun wird eingeengt, der Rückstand in Wasser suspendiert und abfiltriert.

Der Rückstand wird mit 1N Salzsäure, mit Wasser, mit Natriumhydrogencarbonatlösung und wieder mit Wasser gewaschen. Nach dem Trocknen wird ein weißer Festkörper erhalten. (125g = 98% d. Th.)

### B. 3,5-Bis-(N-(3,4-O-isopropyliden-3,4-dihydroxybutyl)-acetamido)-2,4,6-triiodbenzoesäure-N,N-dimethylamid

3,5-Diacetamido-2,4,6-triiodbenzoesäure-N,N-dimethylamid (50g) werden in 2N Natronlauge (200ml) gelöst. Man gibt Dioxan (200ml) und 1,2-O-Isopropyliden-4-O-p-toluolsulfonyl-1,2,4-butantriol (72g) zu. Das Reaktionsgemisch wird 40 Stunden bei 60° bis 70°C gerührt. Nach dem Abkühlen wird eingeengt, mit Wasser versetzt und mehrmals mit Ethylacetat extrahiert. Die organischen Phasen werden getrocknet, eingengt und in Ethylacetat über Kieselgel chromatographiert. (53g = 76% d.Th.)

### C. 3,5-Bis(N-(3,4-dihydroxybutyl)-acetamido)-2,4,6-triiodbenzoesäure-N,N-dimethylamid VII

Man löst 3,5-Bis-(N-(3,4-O-isopropyliden-3,4-dihydroxybutyl)acetamido)-2,4,6-triiodbenzoesäure-N,N-dimethylamid (50g) in Methanol (200ml) gibt 1N Salzsäure (20ml) und Wasser (50ml) zu und läßt 20 Stunden bei Raumtemperatur stehen. Nun wird eingeengt und in Wasser aufgenommen. Die erhaltene Lösung wird über Kohle filtriert und anschließend über eine Anionentauschersäule (200ml Dowex 1 X 2 OH-Form) entsäuert. Nach dem Einengen wird ein weißer Feststoff erhalten. (44g = 96% d. Th.)

### Beispiel 4

### 3,5-Bis(N-(4,5-dihydroxypentyl)-acetamido)-2,4,6-tri iodbenzoesäure-N-methylamid VIII

### A. 3,5-Bis-(N-(4,5-O-isopropyliden-4,5-dihydroxypentyl)-acetamido)-2,4,6-triiodbenzoesäure-N-methylamid

3,5-Diacetamido-2,4,6-triiodbenzoesäure-N-methylamid(50g ) werden in 2N Natronlauge (200ml) gelöst. Man gibt Dioxan (200ml) und 1,2-O-Isopropyliden-5-O-p-toluolsulfonyl-1,2,5-pentantriol (77g) zu. Das Reaktions0-gemisch wird 40 Stunden bei 60° bis 70°C gerührt.

Nach dem Abkühlen wird eingeengt, mit Wasser versetzt und mehrmals mit Ethylacetat extrahiert. Die organischen Phasen werden getrocknet, eingengt und in Ethylacetat über Kieselgel chromatographiert. (55g = 76% d.Th.)

### B. 3,5-Bis(N-(4,5-dihydroxypentyl)-acetamido)-2,4,6-triiodbenzoe-säure-N-methylamid VIII

Man löst 3,5-Bis-(N-(4,5-O-isopropyliden-4,5-dihydroxybutyl)-acetamido)-2,4,6-triiodbenzoesäure-N-methylamid (50g) in Methanol (200ml) gibt 1N Salzsäure (20ml) und Wasser (50ml) zu und läßt 20 Stunden bei Raumtemperatur stehen. Nun wird eingeengt und in Wasser aufgenommen. Die erhaltene Lösung wird über Kohle filtriert und anschließend über eine Anionentauschersäule (200ml Dowex 1 X 2 OH-Form) entsäuert. Nach dem Einengen wird ein weißer Feststoff erhalten. (43g = 94% d. Th.)

### Beispiel 5

### 3,5-Bis(N-(4,5-dihydroxypentyl)-acetamido)-2,4,6-triiodbenzoesäure-N,N-dimethylamid IX

### A. 3,5-Bis-(N-(4,5-O-isopropyliden-4,5-dihydroxypentyl)-acetamido)-2,4,6-triiodbenzoesäure-N,N-dimethylamid

3,5-Diacetamido-2,4,6-triiodbenzoesäure-N,N-dimethylamid (50g) werden in 2N Natronlauge (200ml) gelöst. Man gibt Dioxan (200ml) und 1,2-O-Isopropyliden-5-O-p-toluolsulfonyl-1,2,5-pentantriol (74g)) zu. Das Reaktionsgemisch wird 40 Stunden bei 60° bis 70°C gerührt. Nach dem Abkühlen wird eingeengt, mit Wasser versetzt und mehrmals mit Ethylacetat extrahiert. Die organischen Phasen werden getrocknet, eingengt und in Ethylacetat über Kieselgel chromatographiert. (52g = 72% d.Th.)

### B. 3,5-Bis(N-(4,5-dihydroxypentyl)-acetamido)-2,4,6-triiodbenzoesäure-N,N-dimethylamid IX

Man löst 3,5-Bis-(N-(4,5-O-isopropyliden-4,5-dihydroxybutyl)-acetamido)-2,4,6-triiodbenzoesäure-N,N-dimethylamid (50g) in Methanol (200ml) gibt 1N Salzsäure (20ml) und Wasser (50ml) zu und läßt 20 Stunden bei Raumtemperatur stehen. Nun wird eingeengt und in Wasser aufgenommen. Die erhaltene Lösung wird über Kohle filtriert und anschließend über eine Anionentauschersäule (200ml Dowex 1 X 2 OH-Form) entsäuert. Nach dem Einengen wird ein weißer Feststoff erhalten. (43g = 94% d. Th.)

### Beispiel 6

### N,N'-Dimethyl-N,N'-Bis(3,5-di-(N(4,5-dihydroxybutyl)acetamido)-2,4,6-triiodbenzoyl)-ethylendiamid XV

### A. N,N'-Dimethyl-N,N'-bis-(3,5-diacetamido-2,4,6-triiodbenzoyl)ethylendiamid

Zu einer Lösung von Diatrizoylchlorid (130g) in Dimethylformamid (1l) wird N,N'-Dimethylethylendiamin (8,8g) und Triethylamin (42ml) gegeben. Dieses Gemisch wird 12 Stunden bei 60°C gerührt. Nun wird eingeengt, der Rückstand in Wasser suspendiert und abfiltriert.

Der Rückstand wird mit 1N Salzsäure, mit Wasser, mit Natriumhydrogencarbonatlösung und wieder mit Wasser gewaschen. Nach dem Trocknen wird ein weißer Festkörper erhalten. (118g = 92% d. Th.)

### B. N,N,-Dimethyl-N,N'-Bis(3,5-di-(N(4,5-O-isopropyliden-4,5-dihydroxybutyl)acetamido)-2,4,6-triiodbenzoyl) ethylendiamid.

N,N'-Dimethyl-N,N'-bis-(3,5-diacetamido-2,4,6-triiodbenz oyl)ethylendiamid (25g) werden in 2N Natronlauge (100ml) gelöst. Man gibt Dioxan (100ml) und 1,2-O-Isopropyliden-4-O-p-toluolsulfonyl-1,2,4-butantriol (35g) zu. Das Reaktionsgemisch wird 60 Stunden bei 60° bis 70°C gerührt. Nach dem Abkühlen wird eingeengt, mit Wasser versetzt und mehrmals mit Ethylacetat extrahiert. Die organischen Phasen werden getrocknet, eingengt und in Ethylacetat/Methanol 9:1 über Kieselgel chromatographiert. (24g = 68% d.Th.)

### C. N,N'-Dimethyl-N,N'-Bis(3,5-di-(N(4,5-dihydroxybutyl) acetamido)-2,4,6-triiodbenzoyl)-ethylendiamid XV

Man löst N,N'-Dimethyl-N,N'-Bis(3,5-di-(N(4,5-O-isopropyliden-4,5-dihydroxybutyl)acetamido)-2,4,6-triiodbenzoyl)ethylendiamid (20g) in Methanol (100ml) gibt 1N Salzsäure (10ml) und Wasser (25ml) zu und läßt 20 Stunden bei Raumtemperatur stehen. Nun wird eingeengt und in Wasser aufgenommen. Die erhaltene Lösung wird über Kohle filtriert und anschließend über eine Anionentauschersäule (100ml Dowex 1 X 2 OH-Form) entsäuert. Nach dem Einengen wird ein weißer Feststoff erhalten. (16,6g = 91% d. Th.)

### Beispiel 7

### 5-(N-(3,4-dihydroxybutyl)-acetamido)-2,4,6-triiod-N-methyl-N'-(2,3-dihydroxypropyl)-isophtaldiamid XVII

### A. 5-Acetamido-2,4,6-triiod-N-methyl-N'-(2,3-dihydroxypropyl)isophthaldiamid.

Zu einer Lösung von 5-Acetamido-2,4,6-triiodisophthalsäure-N-methylamid-chlorid (63g) in Dimethylformamid (500ml) wird 3-Amino-1,2-propandiol (10g) und Triethylamin (21ml) gegeben. Dieses Gemisch wird 18 Stunden bei 40°C gerührt. Nun wird eingeengt, der Rückstand in Wasser suspendiert und abfiltriert.

Der Rückstand wird mit 1N Salzsäure, mit Wasser, mit Natriumhydrogencarbonatlösung und wieder mit Wasser gewaschen. Nach dem Trocknen wird ein weißer Festkörper erhalten. (51g = 74% d. Th.)

### B. 5-(N-(3,4-O-isopropyliden-3,4-dihydroxybutyl)-acetamido)-2,4,6-triiod-N-methyl-N'-(2,3-diacetoxypropyl) isophthaldiamid.

5-Acetamido-2,4,6-triiod-N-methyl-N'-(2,3-dihydroxypropyl)-isophthaldiamid (25g) werden in 2N Natronlauge (75ml) gelöst. Man gibt Dioxan (80ml) und 1,2-O-Isopropyliden-4-O-p-toluolsulfonyl-1,2,4-butantriol (17g) zu. Das Reaktionsgemisch wird 20 Stunden bei 50°C gerührt. Nach dem Abkühlen wird mit Salzsäure neutralisiert und zur Trockene eingeengt. Der Rückstand wird in Acetonitril (100ml) suspendiert, mit Acetanhydrid (17ml) und Triethylamin (40ml) versetzt und 24 Stunden bei Raumtemperatur gerührt.

Man gibt Wasser (20ml) zu, läßt eine Stunde stehen und engt ein.

Der Rückstand wird mit Wasser versetzt und mehrmals mit Ethylacetat extrahiert. Die organischen Phasen werden getrocknet, eingeengt und in Ethylacetat über Kieselgel chromatographiert.
(26,5g = 81% d.Th.)

### C. 5-(N-(3,4-dihydroxybutyl)-acetamido)-2,4,6-triiod-N-methyl-N'-(2,3-dihydroxypropyl)-isophthaldiamid XVII

Man löst 5-(N-(3,4-O-isopropyliden-3,4-dihydroxybutyl)-acetamido)-2,4,6-triiod-N-methyl-N'-(2,3-diacetoxypropyl)-isophtaldiamid (25g) in Methanol (100ml) gibt 1N Salzsäure (5ml) und Wasser (25ml) zu und läßt 20 Stunden bei Raumtemperatur stehen. Man gibt 2N Natronlauge (40ml) und läßt weitere 20 Stunden bei Raumtemperatur stehen. Das Methanol wird abdestilliert, die Lösung auf 150ml verdünnt und über Kohle filtriert. Mittels einer Kationentauschersäule (Amberlite CG 120 I, 100ml) und einer Anionentauschersäule (Dowex 1X2, 200ml) wird die Lösung von Salzen befreit. Nach dem Einengen wird ein weißer Feststoff erhalten. (20g = 93% d. Th.)

### Beispiel 8

### N,N'-Dimethyl-N,N'-bis-((5-N-(3,4-dihydroxybutyl)-acetamido)-2,4,6-triiodisophtalsäure-3-(2,3-dihydroxypropyl) amid-ethylendiamid XXIX

### A. 5-Acetamido-2,4,6-triiod-isophtalsäure-N-(2,3-dihydroxypropyl)-amid-chlorid.

Zu einer Lösung von 5-Acetamido-2,4,6-triiod-isophtalsäuredichlorid (50g) in Dimethylformamid (200ml) gibt man Tributylamin (22ml) und eine Lösung von 3-Amino-1,2-propandiol (7,8g) in Dimethylformamid (30ml). Es wird innerhalb von 2 Stunden unter Rühren auf 60°C erwärmt und eine weitere Stunde bei dieser Temperatur gehalten. Nun wird eingeengt, der Rückstand in Methylenchlorid suspendiert und abfiltriert. Das so erhaltene Rohprodukt wird ohne weitere Reinigung für die nächste Stufe eingesetzt.

### B. N,N'-Dimethyl-N,N'-bis-(5-acetamido-2,4,6-triiod-isophthalsäure-3-(2,3-dihydroxypropylamid))-ethylendiamid

Das gesammte Rohprodukt aus Abschnitt A wird in Dimethylformamid (250ml) gelöst, es wird Triethylamin und N,N'-Dimethylethylendiamin (3,4g) zugegeben. Der Ansatz wird bei 60° bis 70°C über Nacht gerührt. Nun wird eingeengt, der Rückstand in wasser suspendiert und abfiltriert. Der Rückstand wird mit 1N Salzsäure, mit Wasser, mit Natriumhydrogencarbonatlösung und wieder mit Wasser gewaschen. Nach dem Trocknen wird ein weißer Festkörper erhalten.
Ausbeute (über Stufen A und B) 31g = 56% d. Th.

### C. N,N'-Dimethyl-N,N'-bis-(5-(N-3,4-O-isopropyliden-3,4-dihydroxybutyl)-acetamido)-2,4,6-triiodisophthalsäure-3-(2,3-diacetoxypropylamid)-ethylendiamid.

N,N'-Dimethyl-N,N'-bis-(5-acetamido-2,4,6-triiodisophthalsäure-3-(2,3-dihydroxypropylamid))-ethylendiamid (25g) werden in 2N Natronlauge (75ml) gelöst. Man gibt Dioxan (80ml) und 1,2-O-Isopropyliden-4-O-p-toluolsulfonyl-1,2,4-butantriol (16g) zu. Das Reaktionsgemisch wird 20 Stunden bei 50°C gerührt. Nach dem Abkühlen wird mit Salzsäure neutralisiert und zur Trockene eingeengt. Der Rückstand wird in Acetonitril (100ml) suspendiert, mit Acetanhydrid (17ml) und Triethylamin (40ml) versetzt und 24 Stunden bei Raumtemperatur gerührt. Man gibt Wasser (20ml) zu, läßt eine Stunde stehen und engt ein. Der Rückstand wird mit Wasser versetzt und mehrmals mit Ethylacetat extrahiert. Die organischen Phasen werden getrocknet, eingeengt und in Ethylacetat über Kieselgel chromatographiert (23,6g = 76% d.Th.)

### D. N,N'-Dimethyl-N,N'-bis-((5-N-(3,4-dihydroxybutyl)-acetamido)-2,4,6-triiodisophtalsäure-3-(2,3-dihydroxypropyl)-amid-ethylendiamid XXVIV

Man löst N,N'-Dimethyl-N,N'-bis-(5-(N-3,4-O-isopropyliden-3,4-dihydroxybutyl)-acetamido)-2,4,6-triiodisophthalsäure-3-(2,3- diacetoxypropylamid) ethylendiamid (23g) in Methanol (100ml) gibt 1N Salzsäure (5ml) und Wasser (25ml) zu und läßt 20 Stunden bei Raumtemperatur stehen. Man gibt 2N Natronlauge (40ml) und läßt weitere 20 Stunden bei Raumtemperatur stehen. Das Methanol wird abdestilliert, die Lösung auf 150ml verdünnt und über Kohle filtriert. Mittels einer Kationentauschersäule (Amberlite CG 120 I, 100ml) und einer Anionentauschersäule (Dowex 1X2, 200ml) wird die Lösung von Salzen befreit. Nach dem Einengen wird ein weißer Feststoff erhalten. (18g = 89% d. Th.)

### Beispiel 9

### 5-(N-(4,5-dihydroxypentyl)-acetamido)-2,4,6-triiod-N-methyl- N'-(2,3-dihydroxypropyl)-isophthaldiamid XIX

### A. 5-(N-(4,5-di-O-acetylpentyl)-acetamido-2,4,6-triiod-N-methyl-isophthalamid

Zu einer Lösung von Natriumhydroxid (1,6g) in Wasser (16ml) wird 5-Acetamido-2,4,6-triiod-N-methylisophthalamid (Iotalaminsäure) (6g) gegeben. Nachdem eine klare Lösung entstanden ist wird eine Lösung von 1,2-O-Isopropyliden-5-O-p-toluolsulfonyl-1,2,5-pentantriol (4,5g) in Dioxan (15ml) zugegeben. Das Reaktionsgemisch wird 24 Stunden bei 60°C gerührt. Nun wird mit Salzsäure angesäuert, das Reaktionsgemisch zur Trockene eingeengt und in Methanol aufgenommen. Von dem nicht gelösten Salzrückstand wird abfiltriert und die Methanollösung wiederum eingeengt. Der erhaltene Rückstand wird in Acetanhydrid (20ml) suspendiert. Nach zweistündigem Erhitzen auf 60°C wird überschüssiges Acetanhydrid im Vakuum abdestilliert. Es verbleiben 7,4g eines rotbraunen Harzes.

### B. 5-N-(4,5-di-O-acetylpentyl)-acetamido)-2,4,6-triiod-N-methylisophthalamidchlorid

Zu einer Suspension von 5-(N-(4,5-di-O-acetylpentyl)-acetamido-2,4,6-triiod-N-methyl-isophthalamid (7g) in Toluol (40ml) gibt man Thionylchlorid (3,5ml) und erhitzt 5 Stunden auf 100°C. Die erhaltene Lösung wird zur Trockene eingeengt. Es werden 7,2g eines rotbraunen Harzes erhalten.

### C. 5-(N-(4,5-di-O-acetylpentyl)-acetamido)-2,4,6-N-methyl-N'-2,3-di-O-acetylpropyl-isophthaldiamid

Zu einer auf 0°C gekühlten Lösung von 3-Aminopropan-1,2-diol (1,3g) und Triethylamin (3ml) in Dimethylformamid (20ml) wird eine Lösung von 5-N-(4,5-di-O-acetylpentyl)-acetamido)-2,4,6-triiod-N-methyl-isophthalamidchlorid (7g) in Dimethylformamid (30ml) getropft. Anschließend wird 15 Stunden bei Raumtemperatur gerührt. Nun wird das Reaktionsgemisch zur Trockene eingeengt und der erhaltene Rückstand in Acetanhydrid suspendiert. Nach Zugabe einer katalytischen Menge Schwefelsäure wird 2 Stunden auf 60°C erwärmt. Das überschüssige Acetanhydrid wird im Vakuum abdestilliert, der Rückstand in Ethylacetat aufgenommen und mit Natriumhydrogencarbonatlösung gewaschen. Die organische Phase wird eingeengt und mit Ethylacetat über Kieselgel chromatographiert. Es werden 5,2g eines hellgelben Feststoffs erhalten.

### D. 5-(N-(4,5-dihydroxypentyl)-acetamido)-2,4,6-triiod-N-methyl-N'-(2,3-dihydroxypropyl)-isophthaldiamid

Eine Lösung von 5-(N-(4,5-di-O-acetylpentyl)-acetamido)-2,4,6-N-methyl-N'-2,3-di-O-acetylpropylisophthaldiamid (3g) in Methanol (30ml) wird mit Kaliumcarbonat (0,4g) 20 Stunden bei Raumtemperatur gerührt. Das Reaktionsgemisch wird zur Trockene eingeengt, der Rückstand in Wasser (30ml) gelöst und mittels einer Kationentauschersäule (Amberlite IR 120) und einer Anionentauschersäule (Dowex 1X2) von ionischen Bestandteilen befreit. Nach dem Einengen verbleiben 2,3g eines farblosen Feststoffes.

## Patentansprüche

1. Verbindungen der allgemeinen Formeln I, II, III und IV 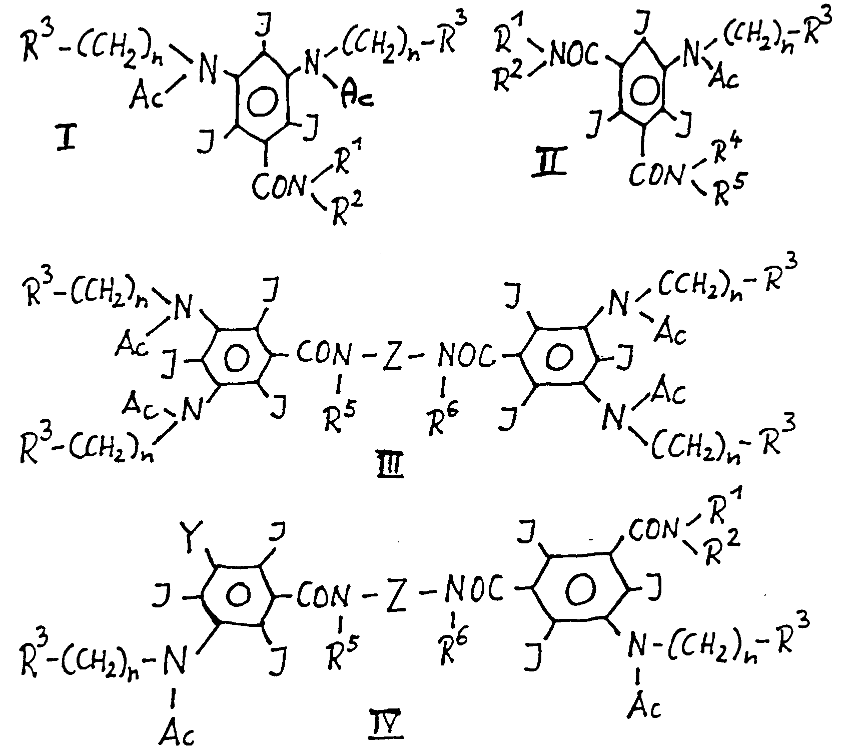 worin Ac einen Acyl-, Hydroxyacyl-, Polyhydroxyacyl- oder Alkoxyacylrest mit 1 bis 4 Kohlenstoffatomen,
R1, R2, R4, R5 und R6 Wasserstoff, einen Alkyl-, Hydroxyalkyl- oder Polyhydroxyalkylrest mit 1 bis 4 Kohlenstoffatomen,
R3 entweder einen linearen Hydroxyalkyl-, Polyhydroxyalkyl-, Hydroxyalkoxy- oder Polyhydroxyalkoxyrest mit 1 bis 4 Kohlenstoffatomen, wobei dann n = 2, 3, 4 ist, oder einen verzweigten Polyhydroxyalkylrest mit 3 bis 6 Kohlenstoffatomen bedeuten, wobei dann n =1, 2, 3 ist und wobei zwischen dem acylierten Stickstoffatom und dem ersten Kohlenstoffatom im Rest R3, das eine Hydroxylgruppe trägt, sich mindestens zwei Kohlenstoffatome befinden, die keine Hydroxylgruppe tragen, und wobei ferner
Z eine direkte Bindung der Stickstoffatome (Hydrazinderivat) oder eine Alkylenkette mit 2 bis 6 Kohlenstoffatomen, die gegebenenfalls durch Sauerstoff unterbrochen sind, und
Y eine der beiden folgenden Gruppen bedeutet:

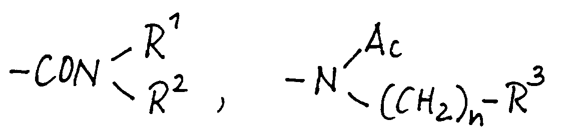

2. 3,5-Bis-(N-(3,4-dihydroxybutyl)-acetamido)-2,4,6-triiodbenzoesäure-N-methylamid VI

3. 3,5-Bis(N-(3,4-dihydroxybutyl)-acetamido)-2,4,6-triiodbenzoesäure-N,N-dimethylamid VII

4. 3,5-Bis(N-(4,5-dihydroxypentyl)-acetamido)-2,4,6-triiodbenzoesäure-N-methylamid VIII

5. 3,5-Bis(N-(4,5-dihydroxypentyi)-acetamido)-2,4,6-triiodbenzoesäure-N,N-dimethylamid IX

6. 3,5-Bis(N-(3,4-dihydroxybutyl)-acetamido)-2,4,6-triiodbenzoe-säure-N-(2-hydroxyethyl)-amid X

7. 3,5-Bis(N-(3,4-dihydroxybutyl)-acetamido)-2,4,6-triiodbenzoesäure-N-(2-hydroxyethyl)-N-methylamid XI

8. 3,5-Bis(N-(4,5-dihydroxypentyl)-acetamido)-2,4,6-triiodbenzoesäure-N-(2-hydroxyethyl)-amid XII

9. 3,5-Bis(N-(4,5-dihydroxypentyl)-acetamido)-2,4,6-triiodbenzoesäure-N-(2-hydroxyethyl)-N-methylamid XIII

10. N'N'-Dimethyl-N,N'-Bis(3,5-di-(N(4,5-dihydroxypentyl)-acetamido)-2,4,6-triiodbenzoyl)-ethylendiamid XIV

11. N,N'-Dimethyl-N,N'-Bis(3,5-di-(N(4,5-dihydroxybutyl) acetamido)-2,4,6-triiodbenzoyl)-ethylendiamid XV

12. N,N'-Bis-(3,5-di-(N-(3,4-dihydroxybutyl)-acetamido)-2,4,6-triiodbenzoyl)-N-(2-hydroxyethyl)-ethylendiamid XVI

13. 5-(N-(3,4-dihydroxybutyl)-acetamido)-2,4,6-triiod-N-methyl-N'-(2,3-dihydroxypropyl)-isophthaldiamid XVII

14. 5-(N-(3,4-dihydroxybutyl)-acetamido)-2,4,6-triiod-N-methyl-N'-(1,3-dihydroxyprop-2-yl)-isophthaldiamid XVIII

15. 5-(N-(4,5-dihydroxypentyl)-acetamido)-2,4,6-triiod-N-methyl-N'-(2,3-dihydroxypropyl)-isophthaldiamid XIX

16. 5-(N-(4,5-dihydroxypentyl)-acetamido)-2,4,6-triiod-N-methyl-N'-(1,3-dihydroxyprop-2-yl)-isophthaldiamid XX

17. 5-(N-(3,4-dihydroxybutyl)-acetamido)-2,4,6-triiod-N-(2-hydroxyethyl)-N'-(2,3-dihydroxypropyl)-isophthaldiamid XXI

18. 5-(N-(3,4-dihydroxybutyl)-acetamido)-2,4,6-triiod-N-(2-hydroxyethyl)-N'-(1,3-dihydroxyprop-2-yl)-isophthaldiamid XXII

19. 5-(N-(4,5-dihydroxypentyl)-acetamido)-2,4,6-triiod-N-(2-hydroxyethyl)-N'-(2,3-dihydroxypropyl)-isophthaldiamid XXIII

20. 5-(N-(4,5-dihydroxypentyl)-acetamido)-2,4,6-triiod-N-(2-hydroxyethyl)-N'-(1,3-dihydroxyprop-2-yl)-isophthaldiamid XXIV

21. 5-(N-(3,4-dihydroxybutyl)-acetamido)-2,4,6-triiod-N,N'-bis-(2-hydroxyethyl)-isophthaldiamid XXV

22. 5-(N-(4,5-dihydroxypentyl)-acetamido)-2,4,6-triiod-N,N'-bis-(2-hydroxyethyl)-isophthaldiamid XXVI

23. 5-(N-(3,4-dihydroxybutyl)-acetamido)-2,4,6-triiod-N,N'-bis-(2-hydroxyethyl)-N,N'-bis-methyl-isophthaldiamid XXVII

24. 5-(N-(4,5-dihydroxypentyl)-acetamido)-2,4,6-triiod-N,N'-bis-(2-hydroxyethyl)-N,N'-bis-methyl-isophthaldiamid XXVIII

25. N,N'-Dimethyl-N,N'-bis-((5-N-(3,4-dihydroxybutyl)-acetamido)-2,4,6-triiodisophthalsäure-3-(2,3-dihydroxypropyl)-amid-ethylendiamid XXIX

26. 3,5-Bis(N-(4,5-dihydroxypentyl)-acetamido)-2,4,6-triiodbenzoesäure-N-ethylamid XXX

27. 3,5-Bis(N-(4,5-dihydroxypentyl)-acetamido)-2,4,6-triiodbenzoesäure-N-propylamid XXXI

28. 3,5-Bis(N-(4,5-dihydroxypentyl)-acetamido)-2,4,6-triiodbenzoesäure-N-isopropylamid XXXII

29. 3,5-Bis(N-(3,4-dihydroxybutyl)-acetamido)-2,4,6-triiodbenzoesäure-N-ethylamid XXXIII

30. 3,5-Bis(N-(3,4-dihydroxybutyl)-acetamido)-2,4,6-triiodbenzoesäure-N-propylamid XXXIV

31. 3,5-Bis(N-(3,4-dihydroxybutyl)-acetamido)-2,4,6-triiodbenzoesäure-N-isopropylamid XXXV

32. 3,5-Bis(N-(5,6-dihydroxyhexyl)-acetamido)-2,4,6-triiodbenzoe-säure-N-methylamid XXXVI

33. 3,5-Bis(N-(5,6-dihydroxyhexyl)-acetamido)-2,4,6-triiodbenzoesäure-N,N-dimethylamid XXXVII

34. 3,5-Bis(N-(3,4-dihydroxybutyl)-acetamido)-2,4,6-triiodbenzoesäureamid XXXVIII

35. 3,5-Bis(N-(4,5-dihydroxypentyl)-acetamido)-2,4,6-triiodbenzoesäureamid XXXIX

36. 3,5-Bis(N-(5,6-dihydroxyhexyl)-acetamido)-2,4,6-triiodbenzoesäureamid XXXX

37. 5-(N-(3,4-dihydroxybutyl)-acetamido)-2,4,6-triiod-N-(2,3-dihydroxypropyl)-isophthaldiamid XXXXI

38. 5-(N-(3,4-dihydroxybutyl)-acetamido)-2,4,6-triiod-N-(1,3-dihydroxyprop-2-yl)-isophthaldiamid XXXXII

39. 5-(N-(4,5-dihydroxypentyl)-acetamido)-2,4,6-triiod-N-(2,3-dihydroxypropyl)-isophthaldiamid XXXXIII

40. 5-(N-(4,5-dihydroxypentyl)-acetamido)-2,4,6-triiod-N-(1,3-dihydroxyprop-2-yl)-isophthaldiamid XXXXIV

41. 3,5-Bis(N-(3-hydroxymethyl-4-hydroxybutyl)-acetamido -2,4,6-triiodbenzoesäure-N-methylamid XXXXV

42. 3,5-Bis(N-(3-hydroxymethyl-4-hydroxybutyl)-acetamido -2,4,6-triiodbenzoesäure-N-ethylamid XXXXVI

43. 3,5-Bis(N-(3-hydroxymethyl-4-hydroxybutyl)-acetamido -2,4,6-triiodbenzoesäure-N-propylamid XXXXVII

44. Verfahren zur Herstellung von Verbindungen nach einem der Ansprüche 1 - 43, dadurch gekennzeichnet, daß man ein 5-Acyl-amido-2,4,6-triiodisophthalsäureamid, ein 3,5-Diacylamido-2,4,6-triiodbenzoesäureamid oder 3,5-Diacylamido-2,4,6-triiodbenzoesäure mit einem Alkylierungsmittel gemäß Formel V in Gegenwart einer geeigneten Base, bevorzugt ein Alkalihydroxid, umsetzt:
X-(CH₂)ₙ - R³ V,
wobei X ein Halogen, ein Sulfonsäureester oder eine übliche Austrittsgruppe bedeutet.

45. Verfahren nach Anspruch 44, dadurch gekennzeichnet, daß als Lösungsmittel Dimethylformamid, Dimethylacetamid, Acetonitril, Dioxan, Tetrahydrofuran, niedere Alkohole, Glycolether, Wasser oder Gemische derselben eingesetzt wird.

46. Verfahren nach Anspruch 44 oder 45, dadurch gekennzeichnet, daß als Abschluß der Synthese etwa vorhandene Hydroxylschutzgruppen abgespalten werden.

47. Röntgenkontrastmittel enthaltend eine Verbindung gemäß einem der Ansprüche 1 bis 43 als Röntgenstrahlen absorbierenden Bestandteil.

## Claims

1. Compounds of the general formulae I, II, III and IV 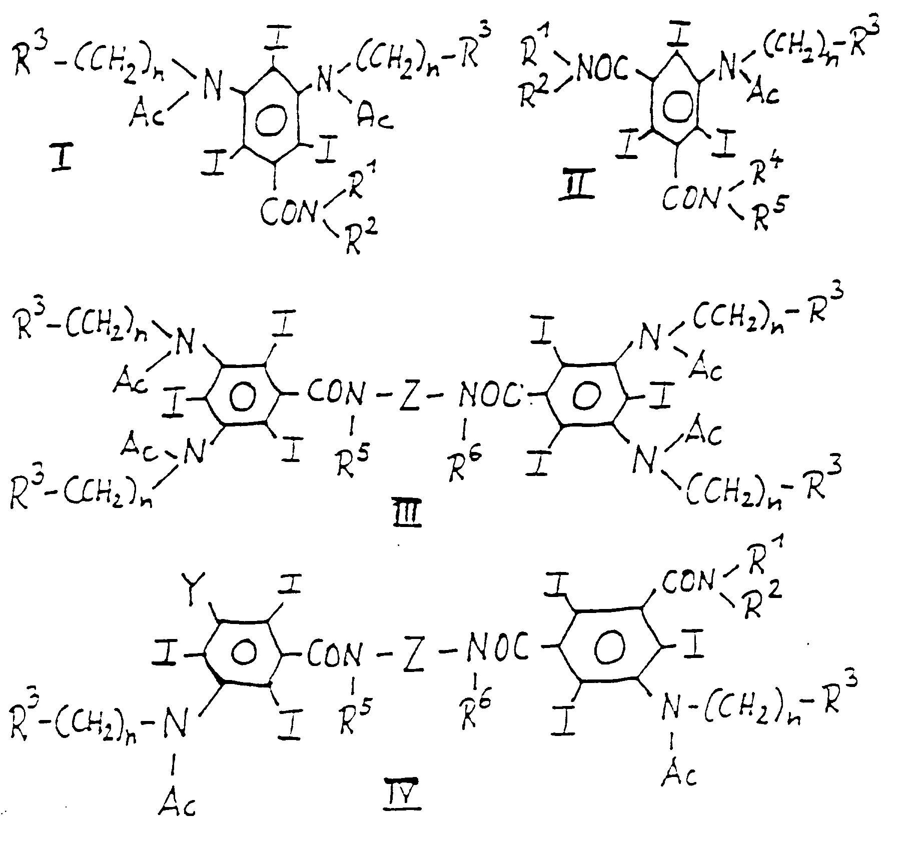 where Ac is an acyl, hydroxyacyl, polyhydroxyacyl or alkoxyacyl residue with 1 to 4 carbon atoms,
R1, R2, R4, R5 and R6 are hydrogen, an alkyl, hydroxyalkyl or polyhydroxyalkyl residue with 1 to 4 carbon atoms,
R3 is either a linear hydroxyalkyl, polyhydroxyalkyl, hydroxyalkoxy or polyhydroxyalkoxy residue with 1 to 4 carbon atoms, in which case n = 2, 3, 4, or a branched polyhydroxyalkyl residue with 3 to 6 carbon atoms, in which case n = 1, 2, 3, and where at least two carbon atoms, not carrying a hydroxyl group, are located between the acylated nitrogen atom and the first carbon atom in the residue R3 which carries a hydroxyl group, and furthermore where
Z is a direct bond between the nitrogen atoms (hydrazine derivative) or an alkene chain with 2 to 6 carbon atoms which are optionally interrupted by oxygen, and
Y is one of the two groups below:

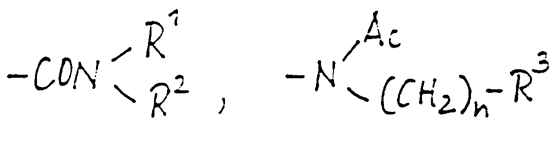

2. 3,5-bis(N-(3,4-dihydroxybutyl)-acetamido)-2,4,6-triiodobenzoic acid-N-methylamide VI.

3. 3,5-bis(N-(3,4-dihydroxybutyl)-acetamido)-2,4,6-triiodobenzoic acid-N,N-dimethylamide VII.

4. 3,5-bis(N-(4,5-dihydroxypentyl)-acetamido)-2,4,6-triiodobenzoic acid-N-methylamide VIII.

5. 3,5-bis(N-(4,5-dihydroxypentyl)-acetamido)-2,4,6-triiodobenzoic acid-N,N-dimethylamide IX.

6. 3,5-bis(N(3,4-dihydroxybutyl)-acetamido)-2,4,6-triiodobenzoic acid-N-(2-hydroxyethyl)-amide X.

7. 3,5-bis(N-(3,4-dihydroxybutyl)-acetamido)-2,4,6-triiodobenzoic acid-N-(2-hydroxyethyl)-N-methylamide XI.

8. 3,5-bis(N-(4,5-dihydroxypentyl)-acetamido)-2,4,6-triiodobenzoic acid-N-(2-hydroxyethyl)-amide XII.

9. 3,5-bis(N-(4,5-dihydroxypentyl)-acetamido)-2,4,6-triiodobenzoic acid-N-(2-hydroxyethyl)-N-methylamide XIII.

10. N,N'-dimethyl-N,N'-bis(3,5-di-(N-(4,5-dihydroxypentyl)-acetamido)-2,4,6-triiodobenzoyl)-ethylene diamide XIV.

11. N,N'-dimethyl-N,N'-bis(3,5-di-(N-(4,5-dihydroxybutyl)-acetamido)-2,4,6-triiodobenzoyl)-ethylene diamide XV.

12. N,N'-bis-(3,5-di-(N-(3,4-dihydroxybutyl)-acetamido)-2,4,6-triiodobenzoyl)-N-(2-hydroxyethyl)-ethylene diamide XVI.

13. 5-(N-(3,4-dihydroxybutyl)-acetamido)-2,4,6-triiodo-N-methyl-N'-(2,3-dihydroxypropyl)-isophthaldiamide XVII.

14. 5-(N-(3,4-dihydroxybutyl)-acetamido)-2,4,6-triiodo-N-methyl-N'-(1,3-dihydroxyprop-2-yl)-isophthaldiamide XVIII.

15. 5-(N-(4,5-dihydroxypentyl)-acetamido)-2,4,6-triiodo-N-methyl-N'-(2,3-dihydroxypropyl)-isophthaldiamide XIX.

16. 5-(N-(4,5-dihydroxypentyl)-acetamido)-2,4,6-triiodo-N-methyl-N'-(1,3-dihydroxyprop-2-yl)-isophthaldiamide XX.

17. 5-(N-(3,4-dihydroxybutyl)-acetamido)-2,4,6-triiodo-N-(2-hydroxyethyl)-N'-(2,3-dihydroxypropyl)-isophthaldiamide XXI.

18. 5-(N-(3,4-dihydroxybutyl)-acetamido)-2,4,6-triiodo-N-(2-hydroxyethyl)-N'-(1,3-dihydroxyprop-2-yl)-isophthaldiamide XXII.

19. 5-(N-(4,5-dihydroxypentyl)-acetamido)-2,4,6-triiodo-N-(2-hydroxyethyl)-N'-(2,3-dihydroxypropyl)-isophthaldiamide XXIII.

20. 5-(N-(4,5-dihydroxypentyl)-acetamido)-2,4,6-triiodo-N-(2-hydroxyethyl)-N'-(1,3-dihydroxyprop-2-yl)-isophthaldiamide XXIV.

21. 5-(N-(3,4-dihydroxybutyl)-acetamido)-2,4,6-triiodo-N,N'-bis-(2-hydroxyethyl)-isophthaldiamide XXV.

22. 5-(N-(4,5-dihydroxypentyl)-acetamido)-2,4,6-triiodo-N,N'-bis-(2-hydroxyethyl)-isophthaldiamide XXVI.

23. 5-(N-(3,4-dihydroxybutyl)-acetamido)-2,4,6-triiodo-N,N'-bis-(2-hydroxyethyl)-N,N'-bis-methyl-isophthaldiamide XXVII.

24. 5-(N-(4,5-dihydroxypentyl)-acetamido)-2,4,6-triiodo-N,N'-bis-(2-hydroxyethyl)-N,N'-bis-methyl-isophthaldiamide XXVIII.

25. N,N'-dimethyl-N,N'-bis-(5-[(]N-(3,4-dihydroxybutyl)-acetamido)-2,4,6-triiodoisophthalic acid-3-(2,3-dihydroxypropyl)-amide[)]-ethylene diamide XXIX.

26. 3,5-bis(N-(4,5-dihydroxypentyl)-acetamido)-2,4,6-triiodobenzoic acid-N-ethylamide XXX.

27. 3,5-bis(N-(4,5-dihydroxypentyl)-acetamido)-2,4,6-triiodobenzoic acid-N-propylamide XXXI.

28. 3,5-bis(N-(4,5-dihydroxypentyl)-acetamido)-2,4,6-triiodobenzoic acid-N-isopropylamide XXXII.

29. 3,5-bis(N-(3,4-dihydroxybutyl)-acetamido)-2,4,6-triiodobenzoic acid-N-ethylamide XXXIII.

30. 3,5-bis(N-(3,4-dihydroxybutyl)-acetamido)-2,4,6-triiodobenzoic acid-N-propylamide XXXIV.

31. 3,5-bis(N-(3,4-dihydroxybutyl)-acetamido)-2,4,6-triiodobenzoic acid-N-isopropylamide XXXV.

32. 3,5-bis(N-(5,6-dihydroxyhexyl)-acetamido)-2,4,6-triiodobenzoic acid-N-methylamide XXXVI.

33. 3,5-bis(N-(5,6-dihydroxyhexyl)-acetamido)-2,4,6-triiodobenzoic acid-N,N-dimethylamide XXXVII.

34. 3,5-bis(N-(3,4-dihydroxybutyl)-acetamido)-2,4,6-triiodobenzoic acid amide XXXVIII.

35. 3,5-bis(N-(4,5-dihydroxypentyl)-acetamido)-2,4,6-triiodobenzoic acid amide XXXIX.

36. 3,5-bis(N-(5,6-dihydroxyhexyl)-acetamido)-2,4,6-triiodobenzoic acid amide XXXX.

37. 5-(N-(3,4-dihydroxybutyl)-acetamido)-2,4,6-triiodo-N-(2,3-dihydroxypropyl)-isophthaldiamide XXXXI.

38. 5-(N-(3,4-dihydroxybutyl)-acetamido)-2,4,6-triiodo-N-(1,3-dihydroxyprop-2-yl)-isophthaldiamide XXXXII.

39. 5-(N-(4,5-dihydroxypentyl)-acetamido)-2,4,6-triiodo-N-(2,3-dihydroxypropyl)-isophthaldiamide XXXXIII.

40. 5-(N-(4,5-dihydroxypentyl)-acetamido)-2,4,6-triiodo-N-(1,3-dihydroxyprop-2-yl)-isophthaldiamide XXXXIV.

41. 3,5-bis(N-(3-hydroxymethyl-4-hydroxybutyl)-acetamido[)]-2,4,6-triiodobenzoic acid-N-methylamide XXXXV.

42. 3,5-bis(N-(3-hydroxymethyl-4-hydroxybutyl)-acetamido[)]-2,4,6-triiodobenzoic acid-N-ethylamide XXXXVI.

43. 3,5-bis(N-(3-hydroxymethyl-4-hydroxybutyl)-acetamido[)]-2,4,6-triiodobenzoic acid-N-propylamide XXXXVII.

44. A process for the preparation of compounds according to any one of claims 1 to 43, characterised in that a 5-acylamido-2,4,6-triiodoisophthalic acid amide, a 3,5-diacylamido-2,4,6-triiodobenzoic acid amide or 3,5-diacylamido-2,4,6-triiodobenzoic acid is reacted with an alkylating agent according to formula V in the presence of a suitable base, preferably an alkali hydroxide:
X-(CH₂)ₙ-R³ V
where X is a halogen, a sulphonic acid ester or a usual leaving group.

45. A process according to claim 44, characterised in that dimethyl formamide, dimethyl acetamide, acetonitrile, dioxan, tetrahydrofuran, low alcohols, glycol ether, water or mixtures thereof are used as a solvent.

46. A process according to claim 44 or 45, characterised in that, as termination of synthesis, existing hydroxyl protecting groups, for example, are separated off.

47. X-ray contrast media containing a compound, according to any one of claims 1 to 43, as an X-ray-absorbing constituent.

## Revendications

1. Composés de formules générales I, II, III et IV 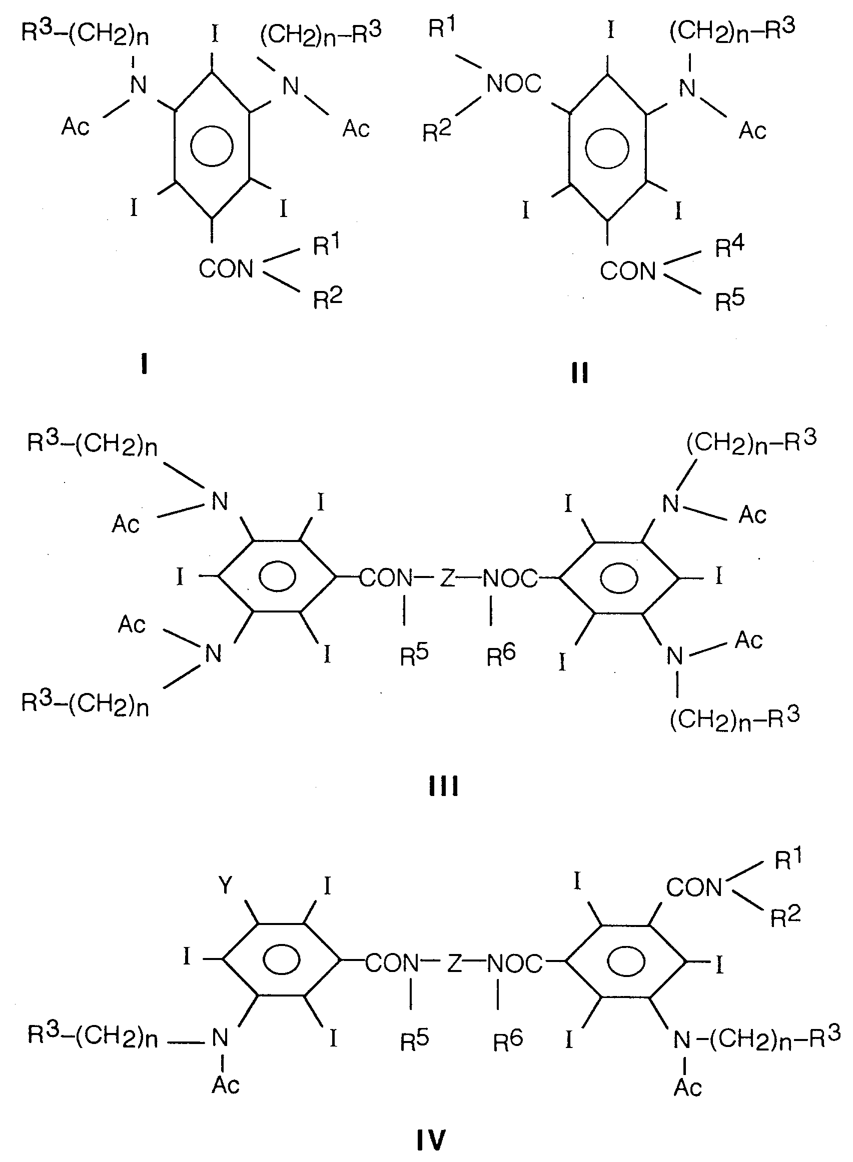 dans lesquelles Ac représente un radical acyle, hydroxyacyle, polyhydroxyacyle ou alkoxyacyle comportant de 1 à 4 atomes de carbone,
R1, R2, R4, R5 et R6 représentent un atome d'hydrogène, ou un radical alkyle, hydroxyalkyle ou polyhydroxyalkyle comportant de 1 à 4 atomes de carbone,
R3 représente soit un radical linéaire hydroxyalkyle, polyhydroxyalkyle, hydroxyalkoxy ou polyhydroxyalkoxy, comportant de 1 à 4 atomes de carbone, dans lequel alors n = 2, 3, 4, soit un radical polyhydroxyalkyle ramifié comportant de 3 à 6 atomes de carbone, dans lequel alors n = 1, 2, 3, et, entre l'atome d'azote acylé et le premier atome de carbone dans le radical R3, qui porte un groupe hydroxy, se trouvent au moins deux atomes de carbone qui ne portent pas de groupe hydroxy, formules dans lesquelles de plus,
Z représente une liaison directe entre les atomes d'azote (dérivé d'hydrazine) ou une chaîne alkylène comportant de 2 à 6 atomes de carbone, qui peut être éventuellement interrompue par l'atome d'oxygène, et
Y représente l'un des deux groupes suivants :

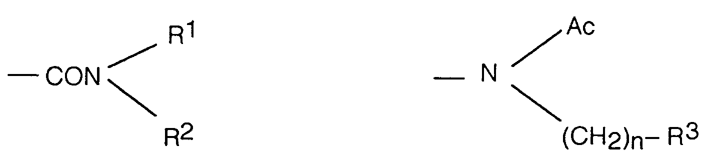

2. N-méthylamide de l'acide 3,5-bis (N-(3,4-dihydroxybutyl)-acétamido)-2,4,6-triiodobenzoïque VI

3. N,N-diméthylamide de l'acide 3,5-bis (N-(3,4-dihydroxybutyl)-acétamido)-2,4,6-triiodobenzoïque VII

4. N-méthylamide de l'acide 3,5-bis (N-(4,5-dihydroxypentyl)-acétamido)-2,4,6-triiodobenzoïque VIII

5. N,N-diméthylamide de l'acide 3,5-bis (N-(4,5-dihydroxypentyl)-acétamido)-2,4,6-triiodobenzoïque IX

6. N-(2-hydroxyéthyl)-amide de l'acide 3,5-bis (N-(3,4-dihydroxybutyl)-acétamido)-2,4,6-triiodobenzoïque X

7. N-(2-hydroxyéthyl)-N-méthylamide de l'acide 3,5-bis (N-(3,4-dihydroxybutyl)-acétamido)-2,4,6-triiodobenzoïque XI

8. N-(2-hydroxyéthyl)-amide de l'acide 3,5-bis (N-(4,5-dihydroxypentyl)-acétamido)-2,4,6-triiodobenzoïque XII

9. N-(2-hydroxyéthyl)-N-méthylamide de l'acide 3,5-bis (N-(4,5-dihydroxypentyl)-acétamido)-2,4,6-triiodobenzoïque XIII

10. N,N'-diméthyl-N,N'-bis (3,5-di-(N(4,5-dihydroxypentyl)-acétamido)-2,4,6-triiodobenzoyl)-éthylènediamide XIV

11. N,N'-diméthyl-N,N'-bis (3,5-di-(N(4,5-dihydroxybutyl)-acétamido)-2,4,6-triiodobenzoyl)-éthylènediamide XV

12. N,N'-bis-(3,5-di-(N-(3,4-dihydroxybutyl)-acétamido)-2,4,6-triiodobenzoyl)-N-(2-hydroxyéthyl)-éthylènediamide XVI

13. 5-(N-(3,4-dihydroxybutyl)-acétamido)-2,4,6-triiodo-N-méthyl-N'-(2,3-dihydroxypropyl)-isophtaldiamide XVII

14. 5-(N-(3,4-dihydroxybutyl)-acétamido)-2,4,6-triiodo-N-méthyl-N'-(1,3-dihydroxypropyl-2)-isophtaldiamide XVIII

15. 5-(N-(4,5-dihydroxypentyl)-acétamido)-2,4,6-triiodo-N-méthyl-N'-(2,3-dihydroxypropyl)-isophtaldiamide XIX

16. 5-(N-(4,5-dihydroxypentyl)-acétamido)-2,4,6-triiodo-N-méthyl-N'-(1,3-dihydroxypropyl-2)-isophtaldiamide XX

17. 5-(N-(3,4-dihydroxybutyl)-acétamido)-2,4,6-triiodo-N-(2-hydroxyéthyl)-N'-(2,3-dihydroxypropyl)-isophtaldiamide XXI

18. 5-(N-(3,4-dihydroxybutyl)-acétamido)-2,4,6-triiodo-N-(2-hydroxyéthyl)-N'-(1,3-dihydroxypropyl-2)-isophtaldiamide XXII

19. 5-(N-(4,5-dihydroxypentyl)-acétamido)-2,4,6-triiodo-N-(2-hydroxyéthyl)-N'-(2,3-dihydroxypropyl)-isophtaldiamide XXIII

20. 5-(N-(4,5-dihydroxypentyl)-acétamido)-2,4,6-triiodo-N-(2-hydroxyéthyl)-N'-(1,3-dihydroxypropyl-2)-isophtaldiamide XXIV

21. 5-(N-(3,4-dihydroxybutyl)-acétamido)-2,4,6-triiodo-N,N'-bis-(2-hydroxyéthyl)-isophtaldiamide XXV

22. 5-(N-(4,5-dihydroxypentyl)-acétamido)-2,4,6-triiodo-N,N'-bis-(2-hydroxyéthyl)-isophtaldiamide XXVI

23. 5-(N-(3,4-dihydroxybutyl)-acétamido)-2,4,6-triiodo-N,N'-bis-(2-hydroxyéthyl)-N,N'-bis-méthyl-isophtaldiamide XXVII

24. 5-(N-(4,5-dihydroxypentyl)-acétamido)-2,4,6-triiodo-N,N'-bis-(2-hydroxyéthyl)-N,N'-bis-méthyl-isophtaldiamide XXVIII

25. 3-(2,3-dihydroxypropyl)-amide-éthylènediamide de l'acide N,N'-diméthyl-N,N'-bis-((5-N-(3,4-dihydroxybutyl)-acétamido)-2,4,6-triiodoisophtalique XXIX

26. N-éthylamide de l'acide 3,5-bis (N-(4,5-dihydroxypentyl)-acétamido)-2,4,6-triiodobenzoïque XXX

27. N-propylamide de l'acide 3,5-bis (N-(4,5-dihydroxypentyl)-acétamido)-2,4,6-triiodobenzoïque XXXI

28. N-isopropylamide de l'acide 3,5-bis (N-(4,5-dihydroxypentyl)-acétamido)-2,4,6-triiodobenzoïque XXXII

29. N-éthylamide de l'acide 3,5-bis (N-(3,4-dihydroxybutyl)-acétamido)-2,4,6-triiodobenzoïque XXXIII

30. N-propylamide de l'acide 3,5-bis (N-(3,4-dihydroxybutyl)-acétamido)-2,4,6-triiodobenzoïque XXXIV

31. N-isopropylamide de l'acide 3,5-bis (N-(3,4-dihydroxybutyl)-acétamido)-2,4,6-triiodobenzoïque XXXV

32. N-méthylamide de l'acide 3,5-bis (N-(5,6-dihydroxyhexyl)-acétamido)-2,4,6-triiodobenzoïque XXXVI

33. N,N-diméthylamide de l'acide 3,5-bis (N-(5,6-dihydroxyhexyl)-acétamido)-2,4,6-triiodobenzoïque XXXVII

34. Amide de l'acide 3,5-bis (N-(3,4-dihydroxybutyl)-acétamido)-2,4,6-triiodobenzoïque XXXVIII

35. Amide de l'acide 3,5-bis (N-(4,5-dihydroxypentyl)-acétamido)-2,4,6-triiodobenzoïque XXXIX

36. Amide de l'acide 3,5-bis (N-(5,6-dihydroxyhexyl)-acétamido)-2,4,6-triiodobenzoïque XXXX

37. 5-(N-(3,4-dihydroxybutyl)-acétamido)-2,4,6-triiodo-N-(2,3-dihydroxypropyl)-isophtaldiamide XXXXI

38. 5-(N-(3,4-dihydroxybutyl)-acétamido)-2,4,6-triiodo-N-(1,3-dihydroxypropyl-2)-isophtaldiamide XXXXII

39. 5-(N-(4,5-dihydroxypentyl)-acétamido)-2,4,6-triiodo-N-(2,3-dihydroxypropyl)-isophtaldiamide XXXXIII

40. 5-(N-(4,5-dihydroxypentyl)-acétamido)-2,4,6-triiodo-N-(1,3-dihydroxypropyl-2)-isophtaldiamide XXXXIV

41. N-méthylamide de l'acide 3,5-bis (N-(3-hydroxyméthyl-4-hydroxybutyl)-acétamido-2,4,6-triiodobenzoïque XXXXV

42. N-éthylamide de l'acide 3,5-bis (N-(3-hydroxyméthyl-4-hydroxybutyl)-acétamido-2,4,6-triiodobenzoïque XXXXVI

43. N-propylamide de l'acide 3,5-bis (N-(3-hydroxyméthyl-4-hydroxybutyl)-acétamido-2,4,6-triiodobenzoïque XXXXVII

44. Procédé de préparation de composés selon l'une des revendications 1 - 43, **caractérisé en ce que** l'on fait réagir un amide de l'acide 5-acyl-amido-2,4,6-triiodoisophtalique, un amide de l'acide 3,5-diacylamido-2,4,6-triiodobenzoïque ou l'acide 3,5-diacylamido-2,4,6-triiodobenzoïque sur un agent d'alkylation selon la formule V, en présence d'une base appropriée, de préférence un hydroxyde alcalin :
X - (CH₂)ₙ - R³ V,
dans laquelle X représente un atome d'halogène, un groupe ester d'acide sulfonique ou un groupe classique susceptible de se séparer.

45. Procédé selon la revendication 44, **caractérisé en ce que** l'on utilise comme solvant le diméthylformamide, le diméthylacétamide, l'acétonitrile, le dioxanne, le tétrahydrofuranne, des alcools inférieurs, un éther de glycol, de l'eau, ou des mélanges de ces solvants.

46. Procédé selon la revendication 44 ou 45, **caractérisé en ce que** comme terminaison de la synthèse, on sépare les groupes protecteurs des groupes hydroxy éventuellement présents.

47. Agent de contraste pour radiographie aux rayons X (Röntgen) contenant un composé selon l'une des revendications 1 à 43 en tant que composant absorbant les rayons X (Röntgen).
